# EUROPÄISCHE PATENTANMELDUNG

(11) **EP 1 726 308 A2**
(43) Veröffentlichungstag der Anmeldung: **29.11.2006**
(21) Anmeldenummer: 06076732.4
(22) Anmeldetag: 22.12.1994
(51) Int. Cl.: A61K 31/57, A61K 31/565, A61P 15/18

(54) **Zusammensetzung für die Empfängnisverhütung umfassend ein Estrogen und ein Gestagen**

(30) Priorität: 22.12.1993 DE 4344462
(62) Teilanmeldung aus: 95905574.0
(71) Anmelder: Schering Aktiengesellschaft, 13342 Berlin (DE)
(72) Erfinder: Spona, Jürgen c/o Vitalogic, 1020 Wien (AT); Düsterberg, Bernd, 16727 Oberkrämer/OT Bärenklau (DE); Lüdicke, Frank, 82024 Taufkrichen (DE); Feichtinger, Wilfried, 1130 Wien (AT)
(74) Vertreter: Plougmann & Vingtoft A/S

(57) **Zusammenfassung**

Die vorliegende Erfindung betrifft ein Monophasisches Kombinationspräparat für die orale Kontrazeption, welches (a) 23 oder 24 Dosierungseinheiten, jeweils enthaltend 0,020 mg Ethinylestradiol und 0,50 bis 0,75 mg Norethisteron; und (b) 5 oder 4 Blindpillen oder andere Indikationen, um anzuzeigen, daß die tägliche Verabreichung der 23 oder 24 Dosierungseinheiten von 5 oder 4 pillenfreien oder Blindpillentagen gefolgt sein soll, umfaßt.

## Beschreibung

Die vorliegende Erfindung betrifft die gemeinsame Verwendung von Estrogenen und Gestagenen zur Herstellung eines monophasischen Kombinationspräparates für die orale Kontrazeption und eine entsprechende, dieses monophasische Kombinationspräparat enthaltende Packung.

Kombinationspraparate zur oralen Kontrazeption sind bereits bekannt, beispielsweise Femovon^{(R)} [DE-PS 2 546 062] oder Morvelon^{(R)} [DE-OS 2 361 120]. Diese Präparate bestehen aus 21 wirkstoffhaltigen (Estrogen/Gestagen) Dosierungseinheiten und 7 wirkstofffreien Dragées (Blindpillen; Placebos). Die taglich zu verabreichende Dosis ist jeweils gleich hoch (sogenannte Einphasen-Präparate) und bewirkt in der gesamten Einnahmezeit und in der Einnahmepause oder während der Einnahme der Placebos den erwünschten kontrazeptiven Effekt. Eine - in den meisten Präparaten - 7tögige Unterbrechung der Einnahme wirkstoffhaltiger Dosierungseinheiten wurde bis vor kurzem für notwendig gehalten, um eine zuverlässige Entzugsblutung auszulosen und damit eine zufriedenstellende Zykluskontrolle zu erreichen.

Andere Präparate, die mehr als 21, einen estrogenen und gestagenen Wirkstoff enthaltende. Dosierungseinheiten aufweisen und in denen die Einnohmepause teilweise (Ijzerman, Pasquale) oder ganz (Kuhl) durch estrogenhaltige Dosierungseinheiten überbrückt wird. Dabei ist es möglich, daß das ansonsten in oralen Kontrazeptiva enthaltene synthetische Estrogen Ethinylestradiol teilweise oder vollständig durch ein konjugiertes Estrogen, vorzugsweise Estradiol, ersetzt ist.

Niedrig dosierte, triphasische Kombinationsprüparate zur hormonalen Kontrazeption, die 21 bis 24 bzw. 24 tägliche Estrogen/Gestagen-Dosierungseinheiten enthalten, gehen bereits aus den EP-As 0491 438 und 0 491 415 hervor. Gemäß der Lehre der EP-A 0 491 438 werden zunächst beginnend mit Tag 1 des Menstruationzyklus die zur Vervollständigung aes im allgemeinen 28 tägigen Zyklus vorgesehenen Placebos eingenommen oder es ist zunächst ein pillenfreies Intervall ohne Einnahme jeglicher kontrazeptiver Steroide vorgesehen.

Ein Kombinationspraparat zur Substitutionstherapie und Kontrazeption für Frauen vor der Menopause (ab etwa dem 40. Lebensjahr) ist aus der EP-A-0 253 607 bekannt. Dieses Kombinationspräparat enthält ein Estrogen aus der Gruppe
17β-Estradiol,
Ethinylestradiol und
Mestranol
sowie ein Gestagen aus der Gruppe
Levonorgestrel,
Gestoden,
Desogestrel,
3-Ketodesogestrel und
Norethindron.

Eine so gewahlte Zusammensetzung soll hormonelle Unregelmaßigkeiten in der Übergangsphase der Praemenopause ausgleichen und die durch die hormonelle Umstellung des weiblichen Organismus in dieser Phase bedingten Beschwerden lindern helfen. Gleichzeitig gewährleistet eine derartige Zusammensetzung einer praemenopausalen Frau den in diesem Lebensalter noch nötigen kontrazeptiven Schutz.

Die Entwicklung neuer oraler Kontrazeptiva für Frauen im fertilen Alter vor der Praemenopause war während der letzten zwanzig Jahre vor allem durch die Reduktion der Estrogen- und Gestagendosierungen gekennzeichnet.

Die Verringerung der täglichen Hormondosis wurde mit der Erwartung verbunden, die Haufigkeit von unerwünschten Nebenwirkungen zu minimieren. Inzwischen erhobene epidemiologische Daten bestätigen den erwünschten Trend zur besseren Verträglichkeit niedriger dosierter Präparate bezogen auf kardiovaskuläre Komplikationen [ (1.) Thorogood M, Oral Contraceptives and Cardiovascular Disease: An Epidemiologic Overview; Pharmacoepidemiology and Drug Safety, Vol 2: 3-16 (1993); (2.) Gerstman B B, Piper J M, Tomita D K, Ferguson W J, Stadel B V, Lundin F E; Oral Contraceptive Estrogen Dose and the Risk of Deep Venous Thromboembolic Disease, Am J.E, Vol. 133, No 1, 32-36 (1991); (3.) Lidegaard O, Oral contraception and rist of a cerebral thromboembolic attack: results of a case-control study; BMJ Vol 306, 956-63 (1993); (4.) Vessey M, Mant D, Smith A, Yeates D., Oral contraceptives and venous thromboembolism: findings in a large prospective study; BMJ, Vol 292, (1986); (5.) Mishell D R, Oral Contraception: Past, Present, and Future Perspectives; Int J Fertil, 36 Suppl., 7 - 18 (1991)].

Es wird angenommen, daß vor allem zwischen der Höhe der Estrogendosis und der inzidenz kardiovaskulärer Erkrankungen ein Zusammenhang besteht. Einer extremen Verringerung der täglichen Estrogendosis steht jedoch der Erhalt der kontrazeptiven Wirksamkeit entgegen. Obwohl die ovulationshemmende Wirkung der niedrig dosierten oralen Kontrazeptiva vorwiegend durch die gestagene Komponente hervorgerufen wird, leistet auch die estrogene Komponente einen erheblichen Beitrag zur zentralen Hemmwirkung und zur ovariellen Suppression (Ovulationshemmung). Darüber hinaus darf die tägliche Estrogendosis Grenzdosisbereiche nicht unterschreiten, damit eine zufriedenstellende Zykluskontrolle gewährleistet werden kann (Der Frauenarzt; 34, 7: 793(1993)).

Die zur Zeit auf dem Markt befindliche niedrigste in einem oralen Kontrazeptivum enthaltene Estrogendosis beträgt 20 µg Ethinylestradiol, kombiniert mit 150 µg Desogestrel (Mercilon). Obwohl die Zykluskontrolle dieses Präparates im Vergleich zu Präparaten mit höherer Estrogendosis erwartungsgemäß etwas schlechter ist, weist die hohe Akzeptanzrate von Mercilon auf eine geringe klinische Relevanz dieses Nachteils hin. Ein klinisch bedeutsames Problem stellt jedoch die in mehreren Studien übereinstimmend gemachte Beobachtung einer geringeren ovoriellen Suppression des 20 µg Ethinylestradiol enthaltenden Präparates dar. Es kommt offensichtlich unter dieser sehr niedrigen Estrogendosis bei vielen Frauen zur Heranreifung von Follikeln, die mit Ultraschalluntersuchungen bzw. Hormonuntersuchungen nachgewiesen werden konnten [(6.) Lunell N O, Carlström K, Zador G, Ovulation inhibition with a combined oral contraceptive containing 20 µg ethinylestradiol and 250 µg levonorgestret; Acta Obstet Gynecol Scand Suppl. 88: 17-21 (1979); (7.) Mall-Haefeli M, Werner-Zodrow I, Huber P R, Klinische Erfahrungen mit Mercilon und Marvelon unter besonderer Berücksichtigung der Ovar-Funktion; Geburtsh, und Frauenheilk. 51, 35-38, Georg Thieme Verlag, Stuttgart-New York (1991); (8.) Strobel E, Behandlung mit oralen Kontrazeptiva; Fortschr. Med. 110 Jg. Nr. 20 (1992); (9.) Letter to Editor, Contraception 45: 519-521 (1992); (10.) Teichmann A T, Brill K, Can Dose Reduction of Ethinylestradiol in OCs jeopardize Ovarian Suppression and Cycle Control? Abstract Book, Vlllth World Congress on Human Reproduction, Bali, Indonesia (1993)].

Die durchgeführten Hormonbestimmungen zeigten, daß es sich um funktionelle Granulosazellen handelt, die 17β-Estradiol sezernieren. Jeder Einnahmefehler bei Frauen mit deutlicher ovarieller Aktivität, also mit Follikelanreifungen, kann zu einem schnellen Anstieg der Gonadotropin-Produktion führen. Die Voraussetzungen für eine Ovulation wären somit vorhanden. Man schätzt, daß etwa ein Drittel der Frauen orale Kontrazeptiva innerhalb eines Anwendungsjahres unregelmäßig einnimmt (Gynpress, 1. Jahrgang, Nr. 3, 1990). Das Risiko einer Schwangerschaft ist deshalb insbesondere bei Einnahmefehlern unter den 20 *µ*g Ethinylestradiol-Präparaten hoch.

Die Aufgabe der vorliegenden Erfindung ist ein verbessertes monophasisches Kombinationspräparat für eine fertile Frau, die sich noch nicht in der Praemenopause befindet, enthaltend in jeder einzelnen Dosierungseinheit ein Estrogen und Gestagen, mit möglichst niedrigem Estrogengehalt in jeder einzelnen Dosierungseinheit, aber auch mit einem niedrigen Gesamthormongehalt pro Verabreichungszyklus.

Es wurde nun gefunden, daß eine ausgeprägte ovarielle Suppression ohne häufige Follikelanfeifungen bei niedriger täglicher Estrogendosierung niedriger Gesamtestrogen- sowie niedriger Gesamthormonmenge pro Verabreichungszyklus erreicht werden kann durch die Verwendung einer Zusammensetzung umfassend ein Estrogen ausgewählt aus
2,0 bis 6,0 mg 17β-Estradiol und
0,015 bis 0,020 mg Ethinylestradiol;
und ein Gestagen ausgewählt aus
0,05 bis 0.075 mg Gestoden,
0,075 bis 0,125 mg Levonorgestrel,
0,06 bis 0,15 mg Desogestrel,
0.06 bis 0,15 mg 3-Ketodesogestrel,
0.1 bis 0,3 mg Drospirenon,
0.1 bis 0,2 mg Cyproteronacetat,
0,2 bis 0,3 mg Norgestimat und
>0,35 bis 0,75 mg Norethisteron
zur Herstellung einer Dosierungsform für die Empfängnisverhütung für eine Frau im fertilen Alter, die die Praemenopause noch nicht erreicht hat, durch Verabreichung der Dosierungsform während 23 oder 24 Tagen, beginnend am Tag eins des Menstruationszyklus (erster Tag der Menstruationsblutung), gefolgt von 5 oder 4 pillenfreien oder Blindpillentagen, während insgesamt 28 Tagen im Verabreichungszyklus.

Die einzelnen Dosierungsformen sollen dabei über alle 23 oder 24 Tage eine konstante Estrogen/Gestagenmenge enthalten.

Die Begriffe "Praemenopause" und "Menopause" werden im Rahmen vorliegender Erfindung im Sinne der konventionellen Definition gebraucht, siehe etwa "The Controversial Climacteric", P.A. von Keep et al., Ed., MTP Press (1981), z.B. S. 9.

Die tägliche Hormondosis wird dabei auf sehr niedrigen Niveau gehalten, während die übliche 21 tägige Einnahme um zwei oder drei Tage verlängert ist. Die verbleibenden 5 oder 4 Tage eines Zyklus werden vorzugsweise durch Placebos überbrückt, um Einnahmefehler zu vermeiden, oder durch 5 oder 4 einnahmefreie Tage.

Gemäß einer bevorzugten Ausführungsform der vorliegenden Erfindung betrifft diese die Verwendung einer Zusammensetzung umfassend ein Estrogen ausgewählt aus
>2,0 bis 6,0 mg 17β-Estradiol und
0.020 mg Ethinylestradiol:
und ein Gestagen ausgewählt aus
>0,06 bis 0,075 mg Gestoden,
>0,100 bis 0,125 mg Levonorgestrel,
>0,10 bis 0,15 mg Desogestrel,
>0.10 bis 0,15 mg 3-Ketodesogestrel,
0,25 bis 0,30 mg Drospirenon,
0,1 bis 0,2 mg Cyproteronacetat,
0.2 bis 0.3 mg Norgestimat und
0,50 bis 0,75 mg Norethisteron
zur Herstellung einer Dosierungsform für die Empfängnisverhütung wie vorstehend beschrieben.

Des weiteren betrifft die vorliegende Erfindung ein monophosisches Kombinations-
produkt für die orale Kontrazeption, welches
(a) 23 oder 24 Dosierungseinheiten, jeweils enthaltend ein Estrogen ausgewählt aus
   >2,0 bis 6,0 mg 17β-Estradiol und
   0.020 mg Ethinylestradiol;
   und ein Gestagen ausgewählt aus
   >0.06 bis 0.075 mg Gestoden,
   >0,100 bis 0,125 mg Levonorgestrel,
   >0,10 bis 0,15 mg Desogestrel,
   >0,10 bis 0,15 mg 3-Ketodesogestrel,
   0,25 bis 0,30 mg Drospirenon,
   0,1 bis 0,2 mg Cyproteronacetat,
   0.2 bis 0,3 mg Norgestimat und
   0.50 bis 0,75 mg Norethisteron
   und
(b) 5 oder 4 Blindpillen oder andere Indikationen, um anzuzeigen, daß die tagliche Verabreichung der 23 oder 24 Dosierungseinheiten von 5 oder 4 pillenfreien oder Blindpillentagen gefolgt sein sollen, umfaßt.

Weitere erfindungsgemäße Ausführungsformen ergeben sich aus den Merkmalen der Unteranspruche.

Ein gemäß der vorliegenden Erfindung besonders bevorzugtes Kombinationspräparat umfaßt 23 Dosierungseinheiten.

iInsbesondere bevorzugt ist ein 23 Dosierungseinheiten aufweisendes monophasisches Kombinationspräparat, enthaltend 20*µ*g Ethinylestradiol und 75 µg Gestoden in jeder Dosierungseinheit und 5 Blindpillen oder andere Indikationen, um anzuzeigen, daß keine Dosierungseinheit oder eine Blindpille während der letzten 5 Tage des Menstruationszyklus verabreicht wird.

Die nachfolgend kurz beschriebene klinische Studie wurde mit Ethinylestradiol als Estrogen und Gestoden als Vertreter der Substanzklasse der erfindungsgemäß möglichen Gestagene durchgeführt.

Die 23tägige Gabe von 20 µg Ethinylestradiol in Kombination mit 75 µg Gestoden führt im Vergleich zur 21 tägigen Gabe zu einer stärkeren ovariellen Suppression. In einer doppelblinden, randomisierten Studie an gesunden Frauen mit normaler Ovarfunktion erhielten Gruppen von je 30 Probandinnen das Kombinationspräparat entweder einmal täglich über 21 oder 23 Tage sowie an 7 bzw. 5 Tagen Placebos (um den Doppelblind-Charakter der Studie zu gewährleisten).

Die Behandlung begann nach einem ovulatorischen, unbehandelten Vorzyklus am 1. Tag der Menstruationsblutung des darauffolgenden Zyklus und erstreckte sich insgesamt über drei Behandlungszyklen. Mit einem unbehandelten follow-up-Zyklus wurde die Studie abgeschlossen.

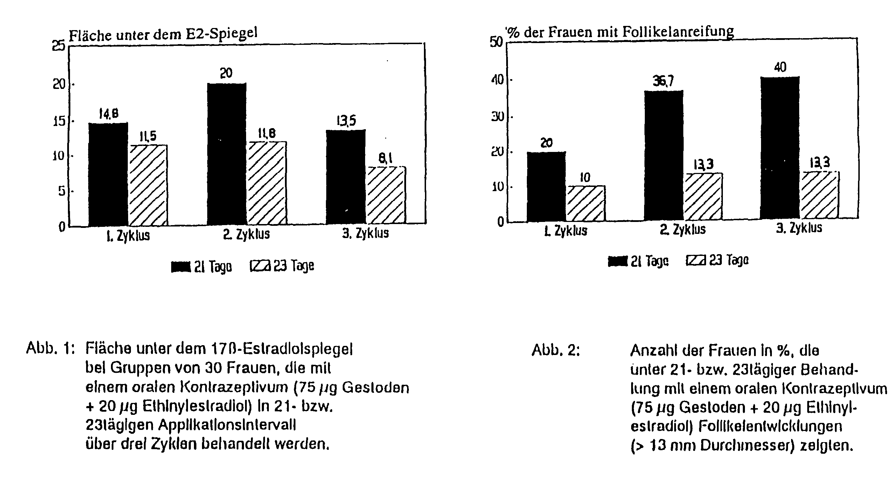

Die ovarielle Suppression wurde anhand der Höhe der endogenen 17β-Estradiolspiegel und der Größe follikularer Strukturen gemessen. Die Ergebnisse zeigen, daß die 17β-Estradiolspiegel unter 23tägiger Einnahme des Prüfpräparates signifikant niedriger (p < 0.05) waren im Vergleich zur 21tägigen Verabreichung (Abb. 1).

In Übereinstimmung mit diesem Befund war auch die Anzahl der Frauen mit Follikelanreifungen deutlich höher bei 21-matiger Gabe gegenüber der 23-maligen Applikation (Abb. 2).

Das lediglich um zwei Tage verlängerte Einnohmeintervoll bewirkt überraschenderweise bei gleichbleibend niedriger Tagesdosis eine signifikant stärkere ovarielle Suppression. Das erfindungsgemäße Kombinationspräparat erreicht somit die bisher für Präparate mit einem täglichen Gehalt von 30 *µ*g Ethinylestradiol bekannte Wirksamkeit, obwohl die tägliche Ethinylestradioldosis um 33 % niedriger und auch die Gesomtdosis pro Zyklus um 27 % geringer ist.

Die Vorteile eines über 23 Tage zu verabreichenden Kombinationspräparates zur oralen Kontrazeption gegenüber den üblichen 21 Tage-Präparaten mit weniger als 30 µg Ethinylestradiol lassen sich wie folgt charakterisieren:
1. Eine signifikant geringere Häufigkeit von Follikelentwicklungen bei der Anwenderin (max. 13% bei Frauen, die das 23 Tage-Präparat gegenüber max. 40% bei denen, die das 21 Tage-Präparat erhielten). Dies bedeutet eine größere kontrazeptive Zuverlassigkeit des 23 Tage-Praparates, insbesondere bei vorhergegangenen Einnahmefehlern, Die Gefahr von "Durchbruchsovulationen" ist geringer.
2. Das Auftreten von großen Follikeln von mehr als 30 mm Durchmesser ist äußerst selten. Die Entwicklung von ovariellen Zysten ist unter dem 23 Tage-Präparat im Vergleich zum 21 Tage-Präparat unwahrscheinlich.
3. Die Rekrutierung von dominanten Follikeln wird in der verkürzten einnahmefreien Pause unterdrückt.
4. Die endogenen 17β-Estradiolspiegel werden beim überwiegenden Teil der Anwenderinnen des 23 Tage-Präparates gut kontrollierbar supprimiert. Klinische Symptome wie Brustspannungen, das prämenstruelle Syndrom und Blutungsstörungen, die auf erhöhte und stark fluktuierende Estrogenspiegel zurückzuführen sind, werden unter dem 23 Tage-Präparat mit deutlich geringerer Häufigkeit beobachtet.

Zusammengefaßt kann eine um zwei (oder drei) Tage verlängerte Einnahme von in jeder täglichen Dosierungseinheit 20 µg Ethinylestradiol enthaltenden Präparaten die genannten Vorteile erbringen, ohne daß die Tages-Dosis auf das bisher weitgehend verwendete Niveau von 30 µg Ethinylestradiol angehoben werden muß.

Vorstehend genannte Vorteile, insbesondere eine bessere Unterdrückung der Follikelanreifung, lassen sich gemäß vorliegender Erfindung mit einem monophasischen Kombinationspräparat erreichen. Gegenüber einem mehrphasischen Präparat zeichnet sich ein monophasisches Präparat durch verschiedene Vorteile aus:
1. leichtere Herstellbarkeit
2. keine Pillenvertauschung aufgrund Nichtbeachtung der Einnahmereihenfolge
3. Menstruationsverschiebungen sind einfacher zu erreichen
4. die Einnahmehinweise sind für die Anwenderin leichter verständlich
5. die Packung bzw. der die Dosierungseinheiten enthaltende Blister braucht nicht mit einem Label zur Beachtung der Einnahmereihenfolge versehen zu sein.

Die Formulierung eines Estrogens und Gestagens für die erfindungsgemaße Verwendung oder für ein erfindungsgemäßes Kombinationspräparat erfolgt vollkommen analog wie es bereits für herkömmmliche orale Kontrazeptiva mit 21tägiger Einnahmedauer der Wirkstoffe wie beispielsweise Femovan^{(R)} (Ethinylestradiol/Gestoden) oder Microgynon^{(R)} (Ethinylestradiol/Levonorgestrel) bekannt ist.

Eine ein erfindungsgemäßes Kombinationbspräparat enthaltende Packung ist ebenfalls analog wie Packungen für bereits bekannte, am Markt befindliche orale Kontrazeptiva aufgebaut mit der Abweichung, daß anstelle der üblichen 21, die aktiven Bestandteile enthaltenden, Dosierungseinheiten,nunmehr 23 oder 24 derartige Dosierungseinheiten und 5 oder 4 Blindpillen vorhanden sind oder aber andere geeignete Hinweise enthalten, daß 5 oder 4 Tage bis zur Fortsetzung der Einnahme wirkstoffhaltiger Dosierungseinheiten zu überbrücken sind.

Im übrigen wird auf die in der EP-A 0 253 607 gemachten Angaben verwiesen, insbesondere auch auf die Angaben dort zur Bestimmung equivalenter Mengen von Ethinylestradiol und 17β-Estrodiol einerseits und verschiedener Gestagene wie Levonorgestrel, Desogestrel, 3-Ketodesogestrel und Gestoden andererseits.

Für weitere Einzelheiten zur Bestimmung von Dosisequivalenten verschiedener gestagener Wirkstoffe wird verwiesen auf "Probleme der Dosisfindung: Sexualhormone"; F. Neumonn et al. in "Arzneimittelforschung" (Drug Research) 27, 2a, 296 - 318 (1977) sowie auf "Aktuelle Entwicklungen in der hormonalen Kontrazeption"; H. Kuhl in "Gynäkologe" 25: 231 - 240 (1992).

## Patentansprüche

1. Monophasisches Kombinationspräparat für die orale Kontrazeption, welches
(a) 23 oder 24 Dosierungseinheiten, jeweils enthaltend 0,020 mg Ethinylestradiol und 0,50 bis 0,75 mg Norethisteron;
und
(b) 5 oder 4 Blindpillen oder andere Indikationen, um anzuzeigen, daß die tägliche Verabreichung der 23 oder 24 Dosierungseinheiten von 5 oder 4 pillenfreien oder Blindpillentagen gefolgt sein soll, umfaßt.

2. Monophasisches Kombinationspräparat nach Anspruch 1, welches 24 Dosierungseinheiten und 4 Blindpillen oder andere indikationen, um anzuzeigen, daß keine Dosierungseinheit oder eine Blindpille während der letzten 4 Tage des Menstruationszyklus verabreicht wird, umfaßt.

3. Monophasisches Kombinationspräparat nach Anspruch 1 oder 2, wobei das Norethisteron in einer Dosis von 0,75 mg vorliegt.
